# EUROPEAN PATENT SPECIFICATION

(11) **EP 4 192 423 B1**
(45) Date of publication and mention of the grant of the patent: **21.05.2025**
(21) Application number: 21759027.2
(22) Date of filing: 28.07.2021
(51) Int. Cl.: A61K 8/49, A61K 8/42, A61K 8/365, A61Q 11/00

(54) **COOLING SENSATION COMPOSITIONS**
KÜHLEMPFINDUNGS-ZUSAMMENSETZUNGEN
COMPOSITIONS DE SENSATION DE REFROIDISSEMENT

(30) Priority: 05.08.2020 GB 202012170
(43) Date of publication of application: 14.06.2023
(73) Proprietor: Givaudan SA, 1214 Vernier (CH)
(72) Inventor: BARTHOLOMEW, Tracy, Kent TN25 7GX (GB); SAJI, Norikazu, Canterbury Kent CT4 7TA (GB); COCITO ARMANINO, Nicolas, 5400 Baden (CH)
(74) Representative: Global Patents
(86) International application number: PCT/EP2021/071109
(87) International publication number: WO 2022/028976

(56) References cited:
- WO-A2-2009/051632
- DE-A1- 102008 015 428
- US-A- 4 190 643

## Description

### TECHNICAL FIELD

This invention is concerned with compositions, and especially solutions comprising at least one cooling compound as further defined herein and a solvent, and the use of said composition in a flavour or fragrance composition.

### BACKGROUND

Compounds providing a cooling sensation have for a long time played an important role in the flavor and fragrance industry in order to produce an association with freshness and cleanliness. Cooling compounds are widely used in a variety of products such as foodstuffs, tobacco products, beverages, dentifrices, mouthwashes, toothpastes, and toiletries. The cooling sensation provided contributed to the appeal and acceptability of consumer products. In particular, oral care products, such as dentifrices and mouthwashes are formulated with coolants because they provide breath freshening effects and a clean, cool, fresh feeling in the mouth.

The newest generation of synthetic cooling compounds is very potent at low concentrations, but unfortunately a lot of them are at room temperature crystalline solids and the use is not without problems. Firstly, it is not always convenient or easy to mix solids into consumer products, which may be in a liquid or a paste-like form. Secondly, powder-like ingredients must be handled with caution to avoid dust hazards associated therewith.

Accordingly, there remains a need to provide cooling compounds in a form that is essay to use in further formulation operations and which remains stable in that form for a long period of storage.

### SUMMARY OF THE INVENTION

In accordance with a first aspect of the present invention there is provided a composition comprising
(a) a compound of formula (I) 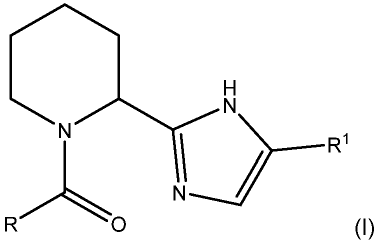 wherein R is an C₃-C₇ branched alkyl or alkenyl optionally comprising one S atom; and R¹ is phenyl, optionally substituted by one or two or three substituents independently selected from the group consisting of Me, F and Cl; and optionally substituted by one further substituent selected from the group consisting of **Et,** vinyl, CN, NO₂, Methoxy and CF₃; and
(b) at least one solvent selected from the butyl lactate, ethanol, ethyl lactate, isopropyl alcohol, isopropylidene glycerol, and lactic acid, or a mixture thereof.

In certain embodiments the solvent is lactic acid.

In accordance with a second aspect of the present invention there is provided a fragrance or flavour composition comprising a composition of the first aspect of the present invention and at least one active selected from the group consisting of flavour and fragrance, and optionally comprising sweetening agent.

In certain embodiments, the at least one flavour / fragrance is selected from a list of ingredients providing additional cooling effect on the skin and/or mucosa, including menthol, mint oil, N-ethyl-p-menthan-3-carboxamide (WS-3), N-(4-(cyanomethyl)phenyl)-2-isopropyl-5-methylcyclohexane-1-carboxamide (Evercool 180), 2-isopropyl-5-methyl-N-(2-(pyridin-2-yl)ethyl)cyclohexane-1-carboxamide (Evercool 190), and menthyl lactate.

In accordance with a third aspect of the present invention there is provided a fragranced or flavoured product comprising a composition of the first aspect of the present invention or a composition of the second aspect of the present invention, and a product base.

In certain embodiments, the product is selected from consumer products which get into contact with the human skin and/or mucosa, including food products, beverages, chewing gum, tobacco and tobacco replacement products, dental care products, personal care products, including lip care products, sexual health and intimate care products.

In certain embodiments, the product is selected from air care products, such as an air freshener or a "ready to use" powdered air freshener which can be used in the home space (rooms, refrigerators, cupboards, shoes or car) and/or in a public space (halls, hotels, malls, etc ..).

As cooling compounds are mainly used in the flavour and fragrance industry, in particular in consumer product which get into contact with the human skin and/or mucosa, solvents which are authorized for the use in such products are particular preferred.

The details, examples and preferences provided in relation to any particular one or more of the stated aspects of the present invention will be further described herein and apply equally to all aspects of the present invention. Any combination of the embodiments, examples and preferences described herein is encompassed by the present invention unless otherwise indicated herein.

### DETAILED DESCRIPTION

The present invention is based, at least in part, on the surprising finding that the cooling compounds of formula (I) 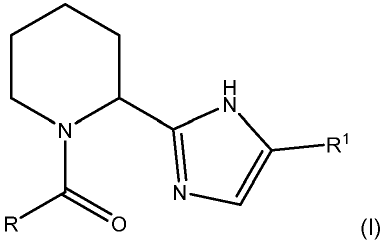 wherein
R is an C₃-C₇ branched alkyl or alkenyl optionally comprising one S atom, and
R' is phenyl, optionally substituted by one or two or three substituents independently selected from the group consisting of CH₃, F and Cl; and optionally substituted by one further substituent selected from the group consisting of Et, vinyl, CN, NO₂, Methoxy and CF₃;
dissolve in higher quantities in a solvent selected from butyl lactate, ethanol, ethyl lactate, isopropyl alcohol, isopropylidene glycerol, and lactic acid, or a mixture thereof.

Non-limiting examples are compounds of formula (I) wherein R¹ is phenyl substituted with one substituent selected from the group consisting of CH₃, F and CI in para position, and optionally substituted by one further substituent selected from the group consisting of Et, vinyl, CN, NO₂, methoxy and CF₃.

In one particular embodiment the compound of formula (I) are represented by the formula (II) 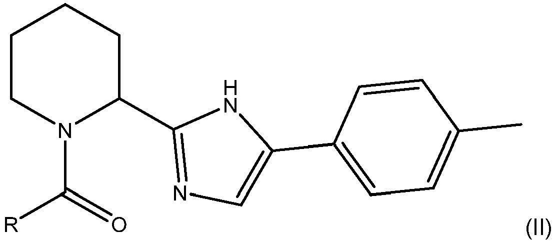 wherein R is an C₃-C₇ branched alkyl or alkenyl optionally comprising one S atom. Thus there is provided in a first aspect a composition comprising a compound of formula (I) and at least one solvent selected from butyl lactate, ethanol, ethyl lactate, isopropyl alcohol, isopropylidene glycerol, and lactic acid, or a mixture thereof.

The compounds of formula (I) as herein defined have been developed by the applicant as new cooling compounds (which are described in more details in PCT/CN2019/111690 patent application of the applicant).

Said compounds are capable to activate the TRPM8 (transient receptor potential melastatin member 8, also known as Trp-p8 or MCR1) ion channels, inducing a sensation of coldness.

The compounds of formula (I) as herein defined are at room temperature crystalline solids, which are relatively insoluble in the common solvents suitable to be used in the flavour and fragrance industry.

Applicant surprisingly found that synthetic cooling compounds, in particular cooling compounds as defined by formula (I), are particular well soluble in a solvent selected from butyl lactate, ethanol, ethyl lactate, isopropyl alcohol, isopropylidene glycerol, and lactic acid, or a mixture thereof.

By "particular well soluble" it is meant within the context of the invention that a stable solution comprising up to 50 weight % of a compound of formula (I) can be prepared.

An advantage of such highly concentrated solutions is that the contribution of an intrinsic odour of the used solvent can be minimised. This is particularly important when such cooling compositions are used in combination with flavour and fragrance formulations. It is also worthwhile to note that the admixture of liquids, e.g., to a flavour / fragrance formulation could be regarded more sustainable than admixing a solid, which would require more energy.

By "stable" it is meant within the context of the invention that no precipitation was observed when stored for up to four weeks at a temperature of at least room temperature (i.e. about 22°C).

Thus there is provided in a further aspect of the present invention a liquid comprising
(a) a compound of formula (I) 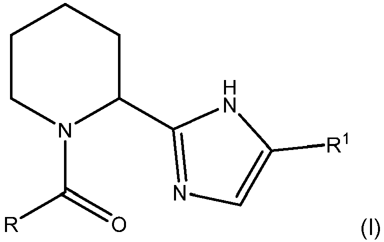 wherein
   R is an C₃-C₇ branched alkyl or alkenyl optionally comprising one S atom, and
   R' is phenyl, optionally substituted by one or two or three substituents independently selected from the group consisting of Me, F and Cl; and optionally substituted by one further substituent selected from the group consisting of Et, vinyl, CN, NO₂, Methoxy and CF₃; and
(b) at least one solvent selected from the butyl lactate, ethanol, ethyl lactate, isopropyl alcohol, isopropylidene glycerol, and lactic acid, or a mixture thereof.

Non-limiting examples are compounds of formula (I) wherein R¹ is phenyl substituted with one substituent selected from the group consisting of CH₃, F and Cl in para position, and optionally substituted by one further substituent selected from the group consisting of Et, vinyl, CN, NO₂, methoxy and CF₃.

In one particular embodiment the compound of formula (I) are represented by the formula (II) 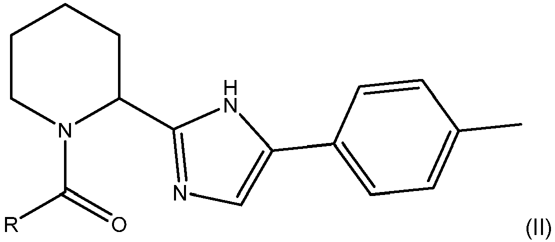 wherein R is an C₃-C₇ branched alkyl or alkenyl optionally comprising one S atom.

In one particular embodiment the liquid comprises at least 3 weight % (e.g. 3 - 50 weight % (e.g., 4 - 45, 5 - 40, 15 - 35, or about 20 weight %) of a compound of formula (I) (which encompasses the compound of formula (II)).

In a further particular embodiment there is provided a liquid composition comprising (a) a compound of formula (I) (which encompasses the compound of formula (II)), and (b) a solvent selected from butyl lactate, ethanol, ethyl lactate, isopropyl alcohol, isopropylidene glycerol, and lactic acid, or a mixture thereof, wherein the weight ratio of ingredient (a) to the solvent (b) to is 1:20 to 1:1 (including 1:18 to 1:2, such as 1:4, 1:3, or 1:5).

In one particular embodiment lactic acid (2-hydroxypropanoic acid) is used as the sole solvent.

Lactic acid is chiral, consisting of two enantiomers. One is known as I-(+)-lactic acid or (S)-lactic acid and the other, its mirror image, is d-(-)-lactic acid or (R)-lactic acid. A mixture of the two in equal amounts is called dl-lactic acid, or racemic lactic acid. dl-Lactic acid is miscible with water and with ethanol above its melting point, which is around 17 °C. Lactic acid is hygroscopic and thus in the food and beverage industry quite often used with a purity of about 85 - 90 weight %. L-Lactic acid is supplemented into foods and beverages (E270), and is widely used as a non-volatile acidulant. The use of the term "lactic acid" in this description encompasses not only the individual enantiomers and the racemate in their pure forms, but also the commercially-available forms of lactic acid, which are generally 85-90 wt % pure.

Even though 50 weight % or even more of a compound of formula (I) (which encompasses the compound of formula (II)) as hereinabove defined are soluble in lactic acid, compositions comprising lower concentrations (e.g. 30 weight % or less, such as 25 - 15 weight %) are preferred due to the viscosity of the resulting mixture. The more viscous a liquid is, the more difficult it is to dose. Instead of reducing the concentration of the compound of formula (I) (which encompasses the compound of formula (II)), healable mixing facilities could be used.

Lactic acid is commercially available, e.g., from PURAC Biochem BV (The Netherlands), and Prinova Europe LTD.

In another particular embodiment lactates (e.g., butyl lactate or ethyl lactate, or a mixture thereof) are used as the solvent.

In another particular embodiment the solvent (b) is selected from butyl lactate, isopropyl alcohol, and isopropylidene glycerol, or a mixture thereof.

The compounds of formula (I) (which encompasses the compound of formula (II)) comprise several chiral centers and as such exist as a mixture of stereoisomers, or they may be resolved as isomerically pure forms. Resolving stereoisomers adds to the complexity of manufacture and purification of these compounds and so it is preferred to use the compounds as mixtures of their stereoisomers simply for economic reasons. However, if it is desired to prepare individual stereoisomers, this may be achieved according to methods known in the art, e.g. preparative HPLC and GC, crystallization or stereoselective synthesis. The compounds as defined by formula (I) (which encompasses the compound of formula (II)) may exist in its tautomeric forms 1 H-Imidazole - 3H-Imidazole form. Accordingly, the chemical structures depicted herein encompass all possible sterioisomers and tautomeric forms of the illustrated compounds.

In one particular embodiment there is provided a liquid composition comprising or consisting of 2-methyl-1-(2-(5-(p-tolyl)-1 H-imidazol-2-yl)piperidin-1-yl)butan-1-one (which includes (2S)-2-methyl-1-(2-(5-(p-tolyl)-1H-imidazol-2-yl)piperidin-1-yl)butan-1-one) and least one solvent selected from the butyl lactate, ethanol, ethyl lactate, isopropyl alcohol, isopropylidene glycerol, and lactic acid, or a mixture thereof.

In a further embodiment there is provided a liquid composition comprising or consisting of 2-methyl-1-(2-(5-(p-tolyl)-1H-imidazol-2-yl)piperidin-1-yl)butan-1-one (which includes (2S)-2-methyl-1-(2-(5-(p-tolyl)-1H-imidazol-2-yl)piperidin-1-yl)butan-1-one) and one solvent selected from the butyl lactate, ethanol, ethyl lactate, isopropyl alcohol, isopropylidene glycerol, and lactic acid, or a mixture thereof.

In another particular embodiment there is provided a composition comprising or consisting of 2-methyl-1-(2-(5-(p-tolyl)-1H-imidazol-2-yl)piperidin-1-yl)butan-1-one (which includes (2S)-2-methyl-1-(2-(5-(p-tolyl)-1H-imidazol-2-yl)piperidin-1-yl)butan-1-one) and lactic acid.

The compositions as defined hereinabove may be added to any products in which a cooling effect on the skin or mucous membrane is desired. It may be employed in the product simply by directly mixing the composition with the product, or it may, in an earlier step, be entrapped in a suitable entrapment material. Alternatively the composition as hereinabove defined may be admixed with other actives, such as, flavours, fragrances, and sweetening agents, and mixtures thereof, before employing it into the product.

Thus there is provided in a further embodiment a flavour or fragrance formulation comprising
(a) a compound of formula (I) 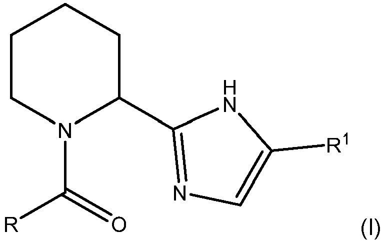
   R is an C₃-C₇ branched alkyl or alkenyl optionally comprising one S atom, and
   R' is phenyl, optionally substituted by one or two or three substituents independently selected from the group consisting of Me, F and Cl; and optionally substituted by one further substituent selected from the group consisting of Et, vinyl, CN, NO₂, Methoxy and CF₃; and
(b) at least one solvent selected from the butyl lactate, ethanol, ethyl lactate, isopropyl alcohol, isopropylidene glycerol, lactic acid, and peppermint oil, or a mixture thereof; and
(c) at least one active selected from the group consisting of flavour and fragrance, and optionally comprising sweetening agent.

In one particular embodiment there is provided a flavour or fragrance formulation comprising
(a) a compound of formula (I) 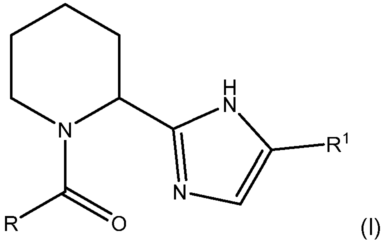
   R is an C₃-C₇ branched alkyl or alkenyl optionally comprising one S atom, and
   R' is phenyl substituted with one substituent selected from the group consisting of CH₃, F and Cl in para position, and optionally substituted by one further substituent selected from the group consisting of Et, vinyl, CN, NO₂, methoxy and CF₃; and
(b) at least one solvent selected from the butyl lactate, ethanol, ethyl lactate, isopropyl alcohol, isopropylidene glycerol, lactic acid, and peppermint oil, or a mixture thereof; and
(c) at least one active selected from the group consisting of flavour and fragrance, and optionally comprising sweetening agent.

In a further particular embodiment there is provided a flavour or fragrance formulation wherein the compound of formula (I) are represented by a compound of formula (II) 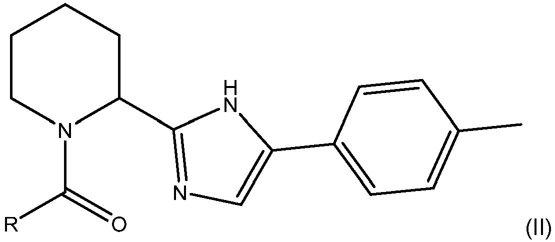 wherein R is an C₃-C₇ branched alkyl or alkenyl optionally comprising one S atom.

Examples of flavour ingredients include natural flavors, artificial flavors, spices, seasonings, and the like. Exemplary flavor ingredients include synthetic flavor oils and flavoring aromatics and/or oils, oleoresins, essences, and distillates, and a combination comprising at least one of the foregoing.

Flavor oils include spearmint oil, cinnamon oil, oil of wintergreen (methyl salicylate), peppermint oil, Japanese mint oil, clove oil, bay oil, anise oil, eucalyptus oil, thyme oil, cedar leaf oil, oil of nutmeg, allspice, oil of sage, mace, oil of bitter almonds, and cassia oil; useful flavoring agents include artificial, natural and synthetic fruit flavors such as vanilla, and citrus oils including lemon, orange, lime, grapefruit, yuzu, sudachi, and fruit essences including apple, pear, peach, grape, raspberry, blackberry, gooseberry, blueberry, strawberry, cherry, plum, prune, raisin, cola, guarana, neroli, pineapple, apricot, banana, melon, apricot, cherry, tropical fruit, mango, mangosteen, pomegranate, papaya, and so forth.

Additional exemplary flavors imparted by a flavoring composition include a milk flavor, a butter flavor, a cheese flavor, a cream flavor, and a yogurt flavor; a vanilla flavor; tea or coffee flavors, such as a green tea flavor, an oolong tea flavor, a tea flavor, a cocoa flavor, a chocolate flavor, and a coffee flavor; mint flavors, such as a peppermint flavor, a spearmint flavor, and a Japanese mint flavor; spicy flavors, such as an asafetida flavor, an ajowan flavor, an anise flavor, an angelica flavor, a fennel flavor, an allspice flavor, a cinnamon flavor, a chamomile flavor, a mustard flavor, a cardamom flavor, a caraway flavor, a cumin flavor, a clove flavor, a pepper flavor, a coriander flavor, a sassafras flavor, a savory flavor, a Zanthoxyli Fructus flavor, a perilla flavor, a juniper berry flavor, a ginger flavor, a star anise flavor, a horseradish flavor, a thyme flavor, a tarragon flavor, a dill flavor, a capsicum flavor, a nutmeg flavor, a basil flavor, a marjoram flavor, a rosemary flavor, a bayleaf flavor, and a wasabi (Japanese horseradish) flavor; a nut flavor such as an almond flavor, a hazelnut flavor, a macadamia nut flavor, a peanut flavor, a pecan flavor, a pistachio flavor, and a walnut flavor; alcoholic flavors, such as a wine flavor, a whisky flavor, a brandy flavor, a rum flavor, a gin flavor, and a liqueur flavor; floral flavors; and vegetable flavors, such as an onion flavor, a garlic flavor, a cabbage flavor, a carrot flavor, a celery flavor, mushroom flavor, and a tomato flavor.

Generally any flavoring or food additive (including food colors) such as those described in "Essential guide to food additives", Third edition2008, page 101 - 321 (ISBN: 978-1-905224-50-0) by Leatherhead Food International Ltd., can be used.

In one particular embodiment the at least one active (c) may be selected from anethole, menthol laevo, carvone laevo, ethyl maltol, vanillin, eucalyptol, eugenol, menthol racemic, cis-3-hexenol, linalol, mint oil (e.g. peppermint arvensis oil, peppermint piperita oil, spearmint native oil, spearmint scotch oil), corylone, ethyl butyrate, cis-3-hexenyl acetate, citral, eucalyptus oil, ethyl-vanillin, methyl salicylate, 2'-hydroxypropiophenone, ethyl acetate, methyl dihydro jasmonate, geraniol, lemon oil, iso amyl acetate, thymol, ionone beta, linalyl acetate, decanal, cis jasmone, ethyl hexanoate, melonal (2,6-dimethylhept-5-enal), citronellol, ethyl aceto acetate, nutmeg oil and clove oil, or mixtures thereof.

In another particular embodiment the at least one active (c) may be selected from menthol (e.g., in form of peppermint oil), menthone, p-menthanecarboxamides, N-2,3-trimethyl-2-isopropyl-butanamide (WS-23), menthyl lactate (Frescolat^{®} ML), menthone glycerol acetal (Frescolat^{®} MGA), 3-(1-menthoxy)-propane-1,2-diol (TK-10), p-menthane-3,8-diol (known as Coolact 38D), isopulegol (known as Coolact P), monomenthyl succinate (Physcool ^{®}), monomenthyl glutarate, o-menthylglycerol, menthyl N,N-dimethylsuccinamate, 2-(sec-butyl)cyclohexan-1-one (Freskomenthe), N-(pyrazol-3-yl)-N-(thiophen-2-ylmethyl)-2-(p-tolyloxy)acetamide, 2-(4-ethylphenoxy)-N-(pyrazol-3-yl)-N-(thiophen-2-ylmethyl)acetamide, 3-(benzo[d][1,3]dioxol-5-yl)-N,N-diphenylacrylamide, 4-(2-(4-allyl-2,6-dimethoxyphenoxy)-1-ethoxypropyl)-2-methoxyphenol, 4-(2-(4-allyl-2,6-dimethoxyphenoxy)-1-((2-isopropyl-5-methylcyclohexyl)oxy)propyl)-2-methoxyphenol (including 4-(2-(4-allyl-2,6-dimethoxyphenoxy)-1-(((1S,2R,5S)-2-isopropyl-5-methylcyclohexyl)oxy)propyl)-2-methoxyphenol) and 4-(2-(4-allyl-2,6-dimethoxyphenoxy)-1-(((1R,2S,5R)-2-isopropyl-5-methylcyclohexyl)oxy)propyl)-2-methoxyphenol), N-(2-Hydroxy-2-phenylethyl)-2-isopropyl-5,5-dimethylcyclohexane-1-carboxamide, N-(4-(Cyanomethyl)phenyl)-2-isopropyl-5,5-dimethylcyclohexanecarboxamide and N-(3-Hydroxy-4-methoxyphenyl )-2-isopropyl-5,5-dimethylcyclohexanecarboxamide.

Examples of p-methanecarboxamides include compounds such as N-ethyl-p-menthan-3-carboxamide (known commercially as WS-3), N-ethoxycarbonylmethyl-p-menthan-3-carboxamide (WS-5), N-( 4-methoxyphenyl)-p-menthan-3-carboxamide (WS-12) and N-tert-butyl-p-menthan-3-carboxamide (WS-14), N-(4-(cyanomethyl)phenyl)-2-isopropyl-5-methylcyclohexane-1-carboxamide (known commercially as Evercool 180), 2-isopropyl-5-methyl-N-(2-(pyridin-2-yl)ethyl)cyclohexane-1-carboxamide (known commercially as Evercool 190), and (1R,2S,5R)-N-((S)-2-((R)-2-aminopropanamido)-2-phenylethyl)-2-isopropyl-5-methylcyclohexane-1-carboxamide.

In one specific embodiment the at least one active (c) is selected from menthol, mint oil, N-ethyl-p-menthan-3-carboxamide (WS-3), N-(4-(cyanomethyl)phenyl)-2-isopropyl-5-methylcyclohexane-1-carboxamide (Evercool 180), 2-isopropyl-5-methyl-N-(2-(pyridin-2-yl)ethyl)cyclohexane-1-carboxamide (Evercool 190), and menthyl lactate.

Examples of sweetening agents include, but are not limited to, sucrose, fructose, glucose, high fructose corn syrup, corn syrup, xylose, arabinose, rhamnose, erythritol, xylitol, mannitol, sorbitol, inositol, acesulfame potassium, aspartame, neotame, sucralose, and saccharine, and mixtures thereof; trilobatin, hesperetin dihydrochalcone glucoside, naringin dihydrochalcone, mogroside V, Luo Han Guo extract, rubusoside, rubus extract, glycyphyllin, isomogroside V, mogroside IV, siamenoside I, neomogroside, mukurozioside Ilb, (+)-hernandulcin, 4 β - hydroxyhernandulcin, baiyunoside, phlomisoside I, bryodulcoside, bryoside bryonoside, abrusosides A-E, cyclocarioside A, cyclocaryoside I, albiziasaponins A-E, glycyrrhizin, araboglycyrrhizin, periandrins I-V, pterocaryosides A and B, osladin, polypodosides A and B, telosmoside A8-18, phyllodulcin, huangqioside E neoastilbin, monatin, 3-acetoxy-5,7-dihydroxy-4'-methoxyflavanone, 2R,3R-(+)-3-Acetoxy-5,7,4'-trihydroxyflavanone, (2R,3R)-dihydroquercetin 3-O-acetate, dihydroquercetin 3-O-acetate 4'-methyl ether, brazzein, curculin, mabinlin, monellin, neoculin, pentadin, thaumatin, and combinations thereof. Some of the compounds listed above are known sweetness enhancers as well as sweeteners. When used as sweetness enhancers they are normally used below their sweetness detection thresholds.

In a further aspect there is provided a flavoured or perfumed product comprising a flavour or fragrance composition and a product base, e.g., an orally acceptable carrier for products which are taken into the mouth, and skin tolerable carriers for products which get into contact with the skin.

In some aspects, the product base may comprise salts (e.g., sodium hydrogen carbonate, sodium carbonate, calcium carbonate, trisodium phosphate, and/or disodium hydrogen phosphate), which may act as a buffer when lactic acid is used as the solvent for the imidazole cooling compound of formula (I) (which encompasses the compound of formula (II)).

In some aspects, the orally acceptable carrier may comprise one or more compatible solid or liquid excipients or diluents which are suitable for topical oral administration. By "compatible," as used herein, is meant that the components of the composition are capable of being commingled without interaction in a manner which would substantially reduce stability and/or efficacy. The carriers can include the usual and conventional components of dentifrices, non-abrasive gels, subgingival gels, mouthwashes or rinses, mouth sprays, chewing gums, lozenges and breath mints. The choice of a carrier to be used is basically determined by the way the composition is to be introduced into the oral cavity. Carrier materials for toothpaste, tooth gel or the like include abrasive materials, sudsing agents, binders, humectants, flavoring and sweetening agents, etc. as disclosed in e.g., U.S. Pat. No. 3,988,433, to Benedict. Carrier materials for biphasic dentifrice formulations are disclosed in U.S. Pat. Nos. 5,213,790; 5,145,666 and 5,281,410 all to Lukacovic et al., and in U. S. Patents 4,849,213 and 4,528,180 to Schaeffer. Mouthwash, rinse or mouth spray carrier materials typically include water, flavoring and sweetening agents, etc., as disclosed in, e.g., U.S. Pat. No. 3,988,433 to Benedict. Lozenge carrier materials typically include a candy base; chewing gum carrier materials include a gum base, flavoring and sweetening agents, as in, e.g., U.S. Pat. No. 4,083,955, to Grabenstetter et al.. Sachet carrier materials typically include a sachet bag, flavoring and sweetening agents. For subgingival gels used for delivery of actives into the periodontal pockets or around the periodontal pockets, a "subgingival gel carrier" is chosen as disclosed in, e.g. U.S. Pat. Nos. 5,198,220 and 5,242,910 both to Damani. Carriers suitable for the preparation of compositions of the present disclosure are well known in the art. Their selection will depend on secondary considerations like taste, cost, and shelf stability, and the like.

Further suitable types of orally acceptable carrier materials or excipients are listed in WO2010/059289, in particular on page 17 - 31.

The composition of the invention may be added to and incorporated into a product base by known methods. Sufficient may be added to provide a cooling effect. The necessary proportion to provide such an effect will naturally depend on the desired cooling effect, but typical weight proportions being from 0.01 to 0.5%. For examples in an oral application of a compound of the present invention, such as dentifrice, floss, chewing gum, or white strip, the levels of use may be from about 0.00001 % (0.01 ppm) to about 0.1 % (1000 ppm); from about 0.00005% (0.5 ppm) to about 0.1 % (1000 ppm); from about 0.0001 % (1 ppm) to about 0.05% (500 ppm); from about 0.005 % (50 ppm) to about 0.03% (300 ppm); or from about 0.001 % (10 ppm) to about 0.01 % (100 ppm) by weight of the composition. When a compound of the present invention is used in a mouthwash, the level of use may be from about 0.000001 % (10 ppb) to about 0.01 % (100 ppm) or from about 0.0001 % (1 ppm) to about 0.001 % (10 ppm) by weight of the composition. When a compound of the present invention is delivered topically, for example in shampoos and lotions the levels may be from about 0.001 % (10 ppm) to about 0.5% (5000 ppm) by weight of the composition or from about 0.01 % (100 ppm) to about 0.4% (4000 ppm) by weight of the composition. These are general guidelines, not rigid boundaries, and formulators seeking particular effects may find it possible or even desirable to formulate outside them.

The composition and methods are now further described with reference to the following non-limiting examples, which describe particular embodiments.

### EXAMPLES

### Example 1: 2-(methylthio)-1-(2-(5-(p-tolyl)imidazol-2-yl)piperidin-1-yl)propan-1-one

Example 1a: *tert-*butyl-2-(1H-imidazol-2-yl)piperidine-1-carboxylate: To a solution of tert-butyl-2-formylpiperidine-1-carboxylate (9.5 g, 35.6 mmol) and glyoxal solution (40% in water, 25.9 g, 178 mmol) in methanol (100 mL) were added dropwise ammonia solution (25 % in water, 17.0 g, 249 mmol) at 0 °C. The solution was allowed to warm up to rt. (room temperature) and stirred at rt. for 16 h. Then the solution was concentrated under reduced pressure, and the result residue was extracted with ethyl acetate (100mL*3). Any precipitate was removed by filtration, and the organic phase was washed with saturated aqueous NaHCO₃ solution (100 mL) and brine (100 mL). The solution was then concentrated under reduced pressure to give tert-butyl-2-(1H-imidazol-2-yl)piperidine-1-carboxylate (5.2 g, yield: 58 %) as white solid. GC/MS (EI): *m*/*z* (%): 251 (3) [M⁺], 195 (4), 178 (10), 150 (20), 134 (13), 122 (5), 95 (100), 82 (10), 57 (21).

Example 1b: *tert*-butyl-2-(4,5-dibromo-1*H*-imidazol-2-yl)piperidine-1-carboxylate: N-bromosuccinimide (7.4 g, 41.8 mmol) was added portionwise to a solution of *tert-*butyl-2-(1H-imidazol-2-yl)piperidine-1-carboxylate (5.0 g, 19.9 mmol) in dichloromethane (100 mL) over 10 min at 0 °C. The mixture was stirred at 0 °C for another 2 h and then concentrated by rotary evaporate. The residue was dissolved in ethyl acetate (250 mL), washed with water (100 mL*2) and brine (100 mL), dried with MgSO₄, and concentrated to get a very brown residue. The residue was recrystallized by dichloromethane/hexanes (1 : 1) to get tert-butyl-2-(4,5-dibromo-1H-imidazol-2-yl)piperidine-1-carboxylate (6.0 g, yield: 74 %) as white solid. GC/MS (EI): *m*/*z* (%): 411 (2) [M⁺], 409 (4) [M⁺], 407 (2) [M⁺], 355 (6), 353 (12), 351 (6), 311 (11), 309 (22), 307 (11), 294 (11), 292 (22), 290 (11), 255 (50), 253 (100), 251 (50), 242 (12), 240 (24), 238 (12), 148 (9), 57 (50).

Example 1c: tert-butyl-2-(5-bromo-1H-imidazol-2-yl)piperidine-1-carboxylate: A suspension of the tert-butyl-2-(4,5-dibromo-1H-imidazol-2-yl)piperidine-1-carboxylate (34.0 g, 90%, 74.8 mmol) and Na₂SO₃ (94g, 748 mmol) in ethanol (300 mL) and water (300 mL) was refluxed overnight. Then cool down and concentrated. The residue was partitioned between CH₂Cl₂ (200 mL) and H₂O (200 mL). The aqueous layer was extracted with ethyl acetate (200 mL*3). The combined organic layers were washed with brine (200 mL), dried with Na₂SO₄, filtered and evaporated. The residue was purified by column chromatography on silica gel to give *tert-butyl-2-*(5-bromo-1H-imidazol-2-yl)piperidine-1-carboxylate (23.0 g, yield: 93 %) as white solid. GC/MS (EI): *m*/*z* (%): 329 (3) [M⁺], 331 (3) [M⁺], 275 (10), 273 (10), 258 (9), 256 (9), 230 (20), 228 (20), 214 (26), 212 (26). 175 (100), 173 (100), 162 (9), 160 (9), 93 (8), 57 (44).

Example 1d: tert-butyl 2-(5-(p-tolyl)-1H-imidazol-2-yl)piperidine-1-carboxylate: A pressure vessel was charged with tert-butyl 2-(5-bromoimidazol-2-yl)piperidine-1-carboxylate (400 mg, 1.211 mmol), p-tolylboronic acid (181 mg, 1.332 mmol, 1.1 equiv.), sodium carbonate (257 mg, 2.42 mmol, 2 equiv.), 1,1'-bis(diphenylphosphino)-ferrocene-palladium(ll)dichloride dichloromethane complex (49 mg, 0.061 mmol, 0.05 equiv.), tetrahydrofuran (5 mL) and water (1 mL). The mixture was degassed by purging with nitrogen and the vessel was sealed. The mixture was stirred and heated to 100°C overnight. The resulting mixture was cooled to 0°C, the vessel was opened and the contents poured into aq. sat. NaHCO₃ solution (50 mL), extracted with EtOAc (2x 50 mL), washed with water (50 mL) and brine (50 mL), dried over MgSO₄ and concentrated under reduced pressure. The crude material was purified by silica gel flash column chromatography eluting with a gradient of EtOAc in Heptane to give tert-butyl 2-(5-(p-tolyl)imidazol-2-yl)piperidine-1-carboxylate (314 mg, 0.920 mmol, 76% yield) as a white solid. MS (El, 70 eV): 341 (4, [M]+•), 285 (11), 268 (3), 240 (30), 185 (100), 172 (16), 91 (6), 57 (99). 1H NMR (DMSO-d6, 400MHz, mixture of rotamers and tautomers): δ 11.66-12.09 (m, 1H), 7.49-7.70 (m, 2H), 7.20-7.48 (m, 1H), 7.08-7.23 (m, 2H), 5.34 - 5.23 (m, 1H), 3.89 (br d, J=12.1 Hz, 1H), 3.05 (br t, J=10.9 Hz, 1H), 2.28 (s, 3H), 2.18-2.25 (m, 1H), 1.65-1.78 (m, 1H), 1.22-1.63 (m, 13H) ppm. ¹³C NMR (75 MHz, DMSO, mixture of tautomers) δ 155.1 (q), 147.5 (q), 140.2 (q), 135.3 (q), 132.7 (q), 129.4 (t), 124.6 (t), 112.5 (t), 79.3 (q), 63.3 (d), 49.7 (t), 41.2 (d), 28.5 (s), 28.4 (d), 26.8 (d), 25.3 (d), 21.2 (s), 19.9 (d) ppm.

Example 1e: 2-(5-(p-tolyl)-1*H*-imidazol-2-yl)piperidine: A solution of tert-butyl 2-(5-(p-tolyl)imidazol-2-yl)piperidine-1-carboxylate (304 mg, 0.890 mmol) in dichloromethane (3 mL) was treated dropwise at 5°C with trifluoroacetic acid (0.549 mL, 7.12 mmol, 8 equiv.) and the resulting mixture stirred at room temperature for 2 hours or until complete consumption of the starting material. The mixture was poured into iced water (30 mL) and the pH made basic by the addition of aqueous 1M NaOH solution. The mixture was then extracted with dichloromethane (3x 20 mL), dried over MgSO₄ and concentrated under reduced pressure to give 2-(5-(p-tolyl)imidazol-2-yl)piperidine (160 mg, 0.664 mmol, 74% yield) as a pale yellow oil which was used in the next step without further purification. MS (El, 70 eV): 241 (6, [M]+•), 185 (100), 172 (13), 158 (8), 91 (3), 84 (4). 1H NMR (CHLOROFORM-d, 400MHz): δ 8.67-8.89 (br s, 1H), 7.50 (d, J=8.1 Hz, 2H), 7.19 (d, J=7.8 Hz, 2H), 7.13 (s, 1H), 4.14 (dd, J=12.3, 3.1 Hz, 1H), 3.30 (br d, J=12.7 Hz, 1H), 2.71-2.84 (m, 1H), 2.37 (s, 3H), 2.15-2.27 (m, 1H), 2.01 (br dd, J=14.4, 3.2 Hz, 1H), 1.90 (br d, J=13.4 Hz, 1H), 1.67-1.77 (m, 2H), 1.32-1.46 ppm (m, 1H).

Example 1f: 2-(methylthio)-1-(2-(5-(p-tolyl)imidazol-2-yl)piperidin-1-yl)propan-1-one: To a solution of 2-(5-(p-tolyl)imidazol-2-yl)piperidine (0.88 mmol) in Dichloromethane (DCM) (2 mL) was added Hydroxybenzotriazole (HOBt) (1.056 mmol, 1.2 equiv.) and 3-(((ethylimino)methylene)amino)-N,N-dimethylpropan-1-amine hydrochloride (EDCl) (1.056 mmol, 1.2 equiv.) at 0-5 °C and the mixture was stirred at room temperature for 0.5 h. The mixture was then treated with 2-(methylthio)propanoic acid (0.968 mmol, 1.1 equiv.) and N,N-diisopropylethylamine (DIPEA) (0.88 mmol, 1 equiv.) and the resulting mixture was stirred at rt. for 16 h. The mixture was filtered and solvent was removed and the crude purified by silica gel chromatography (gradient of EtOAc in Heptane) to furnish 2-(methylthio)-1-(2-(5-(p-tolyl)imidazol-2-yl)piperidin-1-yl)propan-1-one as a white solid.

MS (El, 70 eV): 343 (2, [M]+•), 241 (17), 240 (100), 213 (13), 185 (18), 184 (9), 75 (55), 56 (11), 55 (9), 47 (10), 41 (11). ¹H NMR (400 MHz, DMSO-d₆, mixture of stereoisomers and tautomers) δ 12.07, 11.99, 11.95, 11.76 (brs, 1H), 7.72 - 7.60 (m, 2H), 7.59 - 7.42 (m, 1H), 7.26 - 7.08 (m, 2H), 5.75 - 5.39 (m, 1H), 4.49 - 3.00 (m, 3H), 2.71 - 2.15 (m, 1H), 2.30 (s, 3H), 2.07 - 1.96 (m, 3H), 1.94 - 1.48 (m, 5H), 1.43 - 1.34 (m, 3H) ppm. ¹³C NMR (101 MHz, DMSO-d₆, mixture of stereoisomers and tautomers) δ 170.2 (q), 170.2 (q), 170.0 (q), 147.0 (q), 146.8 (q), 146.7 (q), 140.4 (q), 140.3 (q), 139.9 (q), 135.3 (q), 135.2 (q), 135.1 (q), 132.7 (q), 132.6 (q), 132.6 (q), 129.7 (t), 129.3 (t), 124.6 (t), 113.0 (t), 112.6 (t), 112.5 (t), 51.5 (t), 47.3 (t), 47.0 (t), 43.1 (d), 43.0 (d), 38.8 (d), 38.0 (t), 37.6 (t), 37.3 (t), 28.8 (d), 28.6 (d), 28.1 (d), 27.9 (d), 26.1 (d), 25.7 (d), 25.4 (d), 21.2 (s), 20.3 (d), 20.1 (d), 18.3 (s), 18.0 (s), 17.8 (s), 11.9 (s), 11.7 (s), 11.6 (s) ppm.

### Example 2: 2,2-dimethyl-1-(2-(5-(p-tolyl)-1H-imidazol-2-yl)piperidin-1-yl)but-3-en-1-one

Following the general procedure described in Example 1f: 2,2-dimethylbut-3-enoic acid (170 mg, 1.492 mmol), HOBt (228 mg, 1.492 mmol), 3-(((ethylimino)methylene)amino)-N,N-dimethylpropan-1-amine (232 mg, 1.492 mmol), 2-(5-(p-tolyl)-1H-imidazol-2-yl)piperidine (300 mg, 1.243 mmol) and DIPEA (0.391 ml, 2.238 mmol) in dichloromethane (30 mL) were reacted to give the title product (255 mg, yield: 68 %) as white solid.

GC/MS (EI): *m*/*z* (%): 337 (1) [M⁺], 322 (3), 268 (10), 240 (89), 213 (6), 197 (6), 172 (100), 117(8), 69 (8). ¹H NMR (300 MHz, DMSO-*d*₆, mixture of tautomers) δ 12.04 - 11.70 (m, 1H), 7.83 - 7.51 (m, 2H), 7.51 - 7.37 (m, 1H), 7.32 - 7.00 (m, 2H), 6.23 - 6.10 (m, 1H), 5.81 - 5.34 (m, 1H), 5.27 - 4.80 (m, 2H), 4.60 - 2.96 (m, 2H), 2.43 - 2.17 (m, 4H), 1.87 - 1.43 (m, 5H), 1.40 - 1.19 (m, 6H). ¹³C NMR (75 MHz, DMSO-*d*₆, mixture of tautomers) *δ* 174.1 (q), 147.0 (q), 144.4 (t), 139.9 (q), 135.2 (q), 132.7 (q), 129.7 (t), 129.3 (t), 124.5 (t), 112.9 (t), 112.5 (d), 52.7 (t), 47.6 (t), 45.1 (s), 43.9 (d), 28.0 (d), 27.4 (s), 27.1 (s), 25.3 (d), 21.2 (s), 20.2 (d) ppm.

### Example 3: 2-methyl-1-(2-(5-(p-tolyl)-1H-imidazol-2-yl)piperidin-1-yl)butan-1-one

Example 3a: 2-methyl-1-(2-(5-(p-tolyl)-1H-imidazol-2-yl)piperidin-1-yl)but-3-en-1-one: To a solution of 2-methylbut-3-enoic acid (0.597 g, 5.97 mmol) in dichloromethane (100 mL) was added HOBt (0.914 g, 5.97 mmol) and 3-(((ethylimino)methylene)amino)-N,N-dimethylpropan-1-amine (0.926 g, 5.97 mmol) at 0~5 °C and the mixture was stirred at room temperature for 0.5 h. Then 2-(5-(p-tolyl)-1H-imidazol-2-yl)piperidine (1.2 g, 4.97 mmol) and DIPEA (1.563 ml, 8.95 mmol) was added and the mixture was stirred at rt. for 16 h. The suspension was filtered and solvent was removed and the crude product was purified by silica gel chromatography (hexane : MTBE = 3 : 1) to give 2-methyl-1-(2-(5-(p-tolyl)-1H-imidazol-2-yl)piperidin-1-yl)but-3-en-1-one (808 mg, yield: 50 %) as white solid.

GC/MS (EI, mixture of stereoisomers, ratio 1:2): isomer 1: *m*/*z* (%):323 (2) [M⁺], 268 (5), 240 (100), 172 (95), 117 (7), 84 (3), 55 (11). isomer 2: *m*/*z* (%):323 (2) [M⁺], 268 (5), 240 (100), 211 (6), 172 (81), 117 (7), 84 (2), 55 (10). ¹H NMR (300 MHz, DMSO-d₆, mixture of stereoisomers and tautomers) δ 12.06, 11.81 (brs, 1H), 7.69 - 7.62 (m, 2H), 7.49 (s, 1H), 7.15 (d, *J* = 7.3 Hz, 2H), 6.12 - 5.70 (m, 2H), 5.47 - 4.81 (m, 2H), 4.63 - 2.90 (m, 3H), 2.81 - 2.35 (m, 1H), 2.29 (s, 3H), 1.78 - 1.29 (m, 5H), 1.17 (t, *J =* 5.6 Hz, 3H). ¹³C NMR (75 MHz, DMSO-*d*₆, mixture of stereoisomers and tautomers) δ 172.7 (q), 172.5 (q), 172.3 (q), 147.1 (q), 146.7 (q), 140.3 (q), 139.8 (q), 139.6 (t), 139.1 (t), 135.3 (q), 132.7 (q), 129.4 (t), 124.6 (t), 115.4 (d), 115.3 (d), 113.0 (t), 112.6 (t), 51.7 (t), 51.3 (t), 47.0 (t), 46.8 (t), 42.8 (d), 42.6 (d), 40.2 (t), 39.3 (t), 38.6 (d), 28.4 (d), 28.1 (d), 26.1 (d), 25.8 (d), 25.3 (d), 21.2 (s), 20.3 (d), 20.1 (d), 18.4 (s), 18.2 (s), 18.0 (s) ppm.

Example 3b: 2-methyl-1-(2-(5-(p-tolyl)-1H-imidazol-2-yl)piperidin-1-yl)butan-1-one: 2-methyl-1-(2-(5-(p-tolyl)-1*H*-imidazol-2-yl)piperidin-1-yl)but-3-en-1-one (350 mg, 1.082 mmol) was hydrogenated, catalysed by 10% Pd/C (216 mg, 0.216 mmol) in ethyl acetate (20 ml) under hydrogen atmosphere overnight. The mixture was then purged with nitrogen, filtered over celite and evaporated to give a crude material which was purified by flash column chromatography (hexane : MTBE = 3 : 1) to give the title product (290 mg, yield: 82 %) as white solid.

GC/MS (EI): *m*/*z* (%): 325 (10) [M⁺], 268 (2), 240 (100), 224 (3), 185 (10), 159(2), 142 (1), 84 (2), 57 (4). ¹H NMR (300 MHz, DMSO-*d*₆, mixture of stereoisomers and tautomers) δ 12.05 - 11.72 (m, 1H), 7.73 - 7.53 (m, 2H), 7.51 - 7.40 (m, 1H), 7.27 - 7.03 (m, 2H), 5.81 - 5.28 (m, 1H), 4.61 - 3.16 (m, 2H), 2.87 - 2.58 (m, 1H), 2.38 - 2.19 (m, 4H), 1.86 - 1.49 (m, 5H), 1.46 - 1.23 (m, 2H), 1.09 - 0.95 (m, 3H), 0.94 - 0.80 (m, 3H). ¹³C NMR (75 MHz, DMSO-*d*₆, mixture of stereoisomers and tautomers) δ 175.4 (q), 175.2 (q), 147.3 (q), 147.2 (q), 140.5 (q), 140.1 (q), 135.2 (q), 132.7 (q), 129.7 (t), 129.3 (t), 124.6 (t), 112.9 (t), 112.6 (t), 51.5 (t), 46.9 (t), 46.8 (t), 42.6 (d), 38.6 (d), 36.8 (t), 36.5 (t), 36.1 (t), 29.2 (d), 28.9 (d), 28.4 (d), 27.2 (d), 26.9 (d), 26.2 (d), 25.4 (d), 25.2 (d), 21.2 (s), 20.3 (d), 18.4 (s), 17.7 (s), 17.3 (s), 12.2 (s), 12.0 (s), 11.9 (s) ppm.

### Example 4: 2-methyl-2-(methylthio)-1-(2-(5-(p-tolyl)-1H-imidazol-2-yl)piperidin-1-yl)propan-1-one

Following the general procedure described in Example 1f: 2-methyl-2-(methylthio)propanoic acid (0.267 g, 1.989 mmol), HOBt (305 mg, 1.989 mmol), 3-(((ethylimino)methylene)amino)-N,N-dimethylpropan-1-amine (309 mg, 1.989 mmol), 2-(5-(p-tolyl)-1H-imidazol-2-yl)piperidine (400 mg, 1.657 mmol) and DIPEA (0.521 ml, 2.980 mmol) in dichloromethane (30 mL) were reacted to give the title product (287 mg, yield: 48 %) as white solid.

GC/MS (EI): *m*/*z* (%): 357 (5) [M⁺], 342(7), 268 (11), 240 (100), 185 (11), 159 (5), 117 (4), 89 (10). ¹H NMR (300 MHz, DMSO-*d*₆, mixture of tautomers) δ 12.06, 11.82 (brs, 1H), 7.80 - 7.54 (m, 2H), 7.50 (s, 1H), 7.33 - 7.08 (m, 2H), 6.15 - 5.89 (m, 1H), 4.92 - 2.73 (m, 2H), 2.47 - 2.23 (m, 4H), 2.19 - 2.03 (m, 3H), 1.86 - 1.35 (m, 11H). ¹³C NMR (75 MHz, DMSO-*d*₆, mixture of tautomers) δ 171.1 (q), 170.8 (q), 148.3 (q), 146.8 (q), 140.1 (q), 136.1 (q), 135.2 (q), 132.6 (q), 131.6 (q), 129.7 (t), 129.7 (t), 129.3 (t), 124.6 (t), 112.8 (t), 53.5 (t), 48.4 (t), 47.6 (q), 44.4 (d), 29.3 (d), 28.6 (d), 27.6 (s), 25.8 (d), 21.2 (s), 20.2 (d), 12.9 (s) ppm.

### Example A : solubility of compounds of formula (I)

Into a 20ml vial 0.4 g of 2-methyl-1-(2-(5-(p-tolyl)-1H-imidazol-2-yl)piperidin-1-yl)butan-1-one (white solid) have been added followed by the addition of 1.6 g of the respective solvent as indicated in Table 1 below. The vial was warmed to about 50°C with gentle agitation for 1 hour. If 2-methyl-1-(2-(5-(p-tolyl)-1H-imidazol-2-yl)piperidin-1-yl)butan-1-one hasn't dissolved, 2 g of the respective solvent was added (resulting in a composition comprising 10 weight % of a compound of formula (I)), and again gentle agitated for an additional hour at about 50°C. If 2-methyl-1-(2-(5-(p-tolyl)-1H-imidazol-2-yl)piperidin-1-yl)butan-1-one still hasn't dissolved, 4 g of the respective solvent was added (resulting in a composition comprising 5 weight % of a compound of formula (I)), and again gentle agitated for an additional hour at about 50°C.

If 2-methyl-1-(2-(5-(*p*-tolyl)-1*H*-imidazol-2-yl)piperidin-1-yl)butan-1-one has dissolved, the vial was removed from the heat and was allowed to cool to room temperature. If no precipitation was observed the vials haven been placed in the fridge (about 4°C) over night.

If 2-methyl-1-(2-(5-(*p*-tolyl)-1*H*-imidazol-2-yl)piperidin-1-yl)butan-1-one still has not dissolved, the procedure above has been repeated, starting with 0.05g of 2-methyl-1-(2-(5-(p-tolyl)-1H-imidazol-2-yl)piperidin-1-yl)butan-1-one and 0.95 g of the respective solvent as indicated in Table 1 below. If still not dissolved, 1g of the respective solvent was added (resulting in a composition comprising 2.5 weight % of a compound of formula (I))). If still not dissolved further solvent was added. The results are shown in Table 1 below.

**Table 1: Measure of the solubility of 2-methyl-1-(2-(5-(p-tolyl)-1H-imidazol-2-yl)piperidin-1-yl)butan-1-one in different solvents**

| **Solvent** | **@ RT** | **in the fridge** |
|---|---|---|
| Butyl lactate | 10% | no crystals observed |
| Ethanol abs. | 20% | no crystals observed |
| Ethyl lactate | 10% | no crystals observed |
| Isopropyl alcohol | 10% | no crystals observed |
| Isopropylidene glycerol | 10% | no crystals observed |
| Lactic acid^{a) c)} | 20% | no crystals observed |
| Lactic acid^{b) c)} | 20% | no crystals observed |
| Water dest. | < 1% (not soluble) | not evaluated |
| Propylene glycol | 2.5% | no crystals observed |
| Triethyl citrate | 2.5% | no crystals observed |
| Triacetin | 1% | no crystals observed |

| | | |
|---|---|---|
| a) L-Lactic acid; purity: 86.7 weight % b) dl-Lactic acid; purity: 88 weight % c) same results have been observed for the solubility of (2S)-2-methyl-1-(2-(5-(p-tolyl)-1H-imidazol-2-yl)piperidin-1-yl)butan-1-one | | |

As can be seen from the table above,
- at least 20 weight % of 2-methyl-1-(2-(5-(p-tolyl)-1H-imidazol-2-yl)piperidin-1-yl)butan-1-one is soluble in lactic acid or ethanol;
- at least 10 weight % (but less than 20 weight %) of 2-methyl-1-(2-(5-(p-tolyl)-1H-imidazol-2-yl)piperidin-1-yl)butan-1-one is soluble in butyl lactate, ethyl lactate, isopropyl alcohol, or isopropylidene glycerol;
- only 2.5 weight % of 2-methyl-1-(2-(5-(p-tolyl)-1H-imidazol-2-yl)piperidin-1-yl)butan-1-one is soluble in propylene glycol or triethyl citrate (two common solvents suitable for oral care compositions);
- only 1 weight % of 2-methyl-1-(2-(5-(p-tolyl)-1H-imidazol-2-yl)piperidin-1-yl)butan-1-one is soluble in Triacetin.

### Example B: stability of the solutions comprising a compound of Formula (I) over time at different temperature

The compound of Formula 1 was 2-methyl-1-(2-(5-(p-tolyl)-1H-imidazol-2-yl)piperidin-1-yl)butan-1-one (hereinafter "the compound"). The testing procedure with a number of different solvents was as follows:
1. To 0.4g compound was added 1.6g solvent. This corresponds to a 20% solution.
2. This mixture was warmed to 50°C and agitated for 1 hour.
3. If the compound dissolved, the solution was allowed to cool to room temperature, and then if it remained clear, it was stored overnight at 4°C
4. If crystals had developed, a further 2g solvent was added (this now corresponding to a 10% solution) and step 2 were performed.
5. The observations and actions of step 3 were performed.
6. If the 10% combination of step 4 was not a solution, a further 4g solvent was added (this now corresponding to a 5% solution), and step 2 were performed.
7. The observations and actions of step 3 were performed. If crystals had developed, any such solvent was considered unsuitable

Any solvent in which the compound was not crystallising at a 20 weight % solution, the testing procedure was repeated with a higher concentration as follows:
1. To 1g compound was added 1g solvent. This corresponds to a 50% solution.
2. This mixture was warmed to 50°C and agitated for 1 hour.
3. If the compound dissolved, the solution was allowed to cool to room temperature, and then if it remained clear, it was stored overnight at 4°C
4. If crystals had developed, a further 0.5g solvent was added (this now corresponding to a 40% solution) and step 2 were performed.
5. The observations and actions of step 3 were performed.
6. If the 40% combination of step 4 was not a solution, a further 0.83g solvent was added (this now corresponding to a 30% solution), and step 2 were performed.
7. The observations and actions of step 3 were performed.

Table 2 below shows the solvents in which at least 5 weight % of the compound was soluble (see Example A) and the weight proportions and conditions under which they were considered "good" over a range of weeks at room temperature.

**Table 2: evaluation of the stability of a solution over time comprising 2-methyl-1-(2-(5-(p-tolyl)-1H-imidazol-2-yl)piperidin-1-yl)butan-1-one (weight % as indicated)**

| **Solvent** | @ 4°C Overnight | @ RT 4 week |
|---|---|---|
| Butyl lactate | 20% | 10% |
| Ethanol abs. | 30% | 20% |
| Ethyl lactate | 10% | 10% |
| Isopropyl alcohol | 10% | 5% |
| Isopropylidene glycerol | 10% | 5% |
| Lactic acid^{d)f)} | 50% | 50% |
| Lactic acid^{e)f)} | 50% | 50% |

| | | |
|---|---|---|
| d) L-Lactic acid; purity: 86.7 weight % e) dl-Lactic acid; purity: 88 weight % f) same results have been observed for the solubility of (2S)-2-methyl-1-(2-(5-(p-tolyl)-1*H*-imidazol-2-yl)piperidin-1-yl)butan-1-one | | |

As can be seen from Table 2, a composition
a) using lactic acid as the solvent and comprising 50 weight % of a compound of formula (I) is stable, both in the fridge and at room temperature (RT) for at least four weeks;
b) using ethanol as the solvent and comprising 30 weight % of a compound of formula (I) is stable in the fridge;
c) using ethanol as the solvent and comprising 20 weight % of a compound of formula (I) at room temperature (RT) for at least four weeks;
d) using ethyl lactate as the solvent and comprising 10 weight % of a compound of formula (I) is stable , both in the fridge and at room temperature (RT) for at least four weeks.

### Example C: Preparation of a flavor formulation

The flavoring compositions were prepared by admixing the following ingredients:

| Ingredient | parts by weight |
|---|---|
| CLOVE TERPENELESS | 0.3 |
| ETHYL MALTOL | 0.1 |
| 20 wt% solution of Cooling compound* in lactic acid | 1.0 |
| GINGER OLEORESIN | 0.1 |
| LEMON Oil | 0.5 |
| MENTHOL LAEVO EXTRA | 58.0 |
| ORANGE Oil | 0.5 |
| PEPPERMINT Oil | 35.0 |
| PROPYLENE GLYCOL | 0.4 |
| SPEARMINT Oil | 4.0 |
| VANILLIN | 0.1 |
| | 100.0 |

| Ingredient | parts by weight |
|---|---|
| CLOVE TERPENELESS | 1.6 |
| ETHYL MALTOL | 0.1 |
| EUCALYPTUS Oil | 3.0 |
| EUGENOL | 6.0 |
| 20 wt% solution of Cooling compound* in lactic acid | 0.9 |
| MENTHOL LAEVO | 56.0 |
| MENTHOL RACEMIC | 8.0 |
| METHYL SALICYLATE | 18.2 |
| PEPPERMINT Oil | 6.0 |
| TEA TREE PURE | 0.1 |
| TOSCANOL** | 0.1 |
| | 100.00 |

| Ingredient | parts by weight |
|---|---|
| CARVONE LAEVO | 12.0 |
| ETHYL MALTOL | 0.1 |
| 20 wt% solution of Cooling compound* in lactic acid | 1.12 |
| MENTHOL LAEVO | 42.7 |
| PEPPERMINT Oil | 16.0 |
| SPEARMINT Oil | 28.0 |
| VANILLA extract | 0.08 |
| | 100.0 |

| | |
|---|---|
| *: 2-methyl-1-(2-(5-(p-tolyl)-1*H*-imidazol-2-yl)piperidin-1-yl)butan-1-one **: 1-(cyclopropylmethyl)-4-methoxybenzene; origin: Givaudan SA, Vernier, Switzerland | |

The flavoring compositions may be added to an oral care product (e.g. mouth wash) or to confectionary (e.g. chewing gum).

## Claims

1. A composition comprising
a) a cooling compound of formula (I) 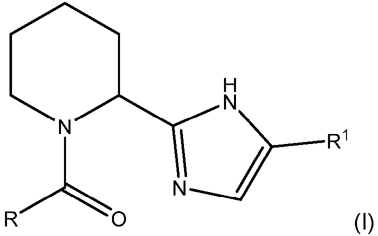 wherein
R is an C₃-C₇ branched alkyl or alkenyl optionally comprising one S atom, and R' is phenyl, optionally substituted by one or two or three substituents independently selected from the group consisting of CH₃, F and Cl; and optionally substituted by one further substituent selected from the group consisting of Et, vinyl, CN, NO₂, Methoxy and CF₃;
b) and at least one solvent selected from butyl lactate, ethanol, ethyl lactate, isopropyl alcohol, isopropylidene glycerol, and lactic acid, or a mixture thereof.

2. A composition according to claim 1, wherein the compound of formula (I) are represented by the formula (II) 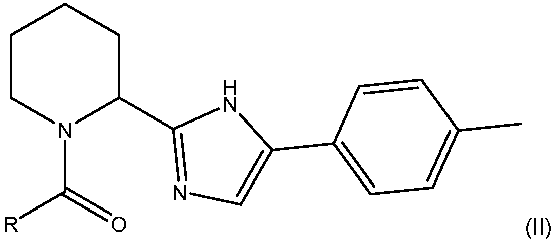 wherein R is an C₃-C₇ branched alkyl or alkenyl optionally comprising one S atom.

3. A composition according to claim 1 or claim 2, wherein the weight ratio of ingredient (a) to the solvent (b) to is 1:20 to 1:1.

4. A composition according to any preceding claim, wherein the composition is a liquid.

5. A composition according to any preceding claim, wherein the cooling compound of formula (I) is 2-methyl-1-(2-(5-(p-tolyl)-1H-imidazol-2-yl)piperidin-1-yl)butan-1-one, 2-(methylthio)-1-(2-(5-(p-tolyl)-1H-imidazol-2-yl)piperidin-1-yl)propan-1-one, 2-methyl-2-(methylthio)-1-(2-(5-(p-tolyl)-1H-imidazol-2-yl)piperidin-1-yl)propan-1-one, or 2,2-dimethyl-1-(2-(5-(p-tolyl)-1H-imidazol-2-yl)piperidin-1-yl)but-3-en-1-one, or a mixture thereof.

6. A composition according to any preceding claim wherein the solvent (b) is lactic acid.

7. A composition according to any preceding claim, **characterized in that** the composition comprises at least 3 weight % of at least one compound of formula (I), relative to the total weight of the composition.

8. A composition according to any preceding claim comprising, consisting essentially of, or consisting of (a) 3 - 50 weight % of 2-methyl-1-(2-(5-(p-tolyl)-1H-imidazol-2-yl)piperidin-1-yl)butan-1-one and (b) 50 - 97 weight % of lactic acid, related to the total mixture, provided that (a) and (b) add up to 100 weight %.

9. A fragrance or flavour formulation comprising a composition as defined in any proceeding claim, further comprising at least one active selected from the group consisting of flavour and fragrance, and optionally comprising sweetening agent.

10. A fragranced or flavoured product, comprising a product base and a cooling effect-providing proportion of a composition according to claim 1, or a formulation according to claim 9.

11. A method of incorporating at least one cooling compound (a) as defined in claim 1 in a fragranced or flavoured product, comprising the blending of (a) at least one cooling compound of formula (I) with at least one solvent selected from butyl lactate, ethanol, ethyl lactate, isopropyl alcohol, isopropylidene glycerol, and lactic acid, or a mixture thereof, and the addition of the resulting composition to the product.

12. A method of providing to a product a cooling sensation for the skin or the mucous membrane, comprising the addition to a product base of a cooling effect-providing proportion of a composition according to any one of claims 1 to 8.

## Patentansprüche

1. Zusammensetzung, umfassend
a) eine kühlende Verbindung der Formel (I) 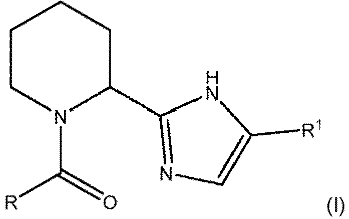 wobei
R für ein verzweigtes C₃-C₇-Alkyl oder -Alkenyl, das gegebenenfalls ein S-Atom enthält, steht und R¹ für Phenyl, das gegebenenfalls durch einen oder zwei oder drei Substituenten, die aus der Gruppe bestehend aus CH₃, F und Cl ausgewählt sind, substituiert ist und gegebenenfalls durch einen weiteren Substituenten, der aus der Gruppe bestehend aus Et, Vinyl, CN, NO₂, Methoxy und CF₃ ausgewählt ist, substituiert ist, steht;
b) und mindestens ein Lösungsmittel, das aus Butyllactat, Ethanol, Ethyllactat, Isopropylalkohol, Isopropylidenglycerin und Milchsäure oder einer Mischung davon ausgewählt ist.

2. Zusammensetzung nach Anspruch 1, wobei die Verbindung der Formel (I) durch die Formel (II) wiedergegeben wird: 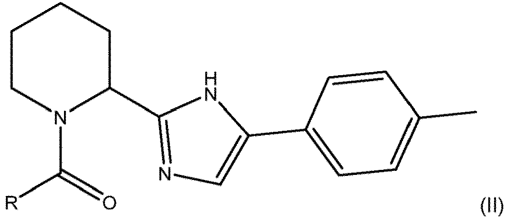 wobei R für ein verzweigtes C₃-C₇-Alkyl oder - Alkenyl, das gegebenenfalls ein S-Atom enthält, steht.

3. Zusammensetzung nach Anspruch 1 oder Anspruch 2, wobei das Gewichtsverhältnis von Bestandteil (a) zu Lösungsmittel (b) 1:20 bis 1:1 beträgt.

4. Zusammensetzung nach einem der vorhergehenden Ansprüche, wobei es sich bei der Zusammensetzung um eine Flüssigkeit handelt.

5. Zusammensetzung nach einem der vorhergehenden Ansprüche, wobei es sich bei der kühlenden Verbindung der Formel (I) um 2-Methyl-1-(2-(5-(p-tolyl)-1H-imidazol-2-yl)piperidin-1-yl)butan-1-on, 2-(Methylthio)-1-(2-(5-(p-tolyl)-1H-imidazol-2-yl)piperidin-1-yl)propan-1-on, 2-Methyl-2-(methylthio)-1-(2-(5-(p-tolyl)-1H-imidazol-2-yl)piperidin-1-yl)propan-1-on oder 2,2-Dimethyl-1-(2-(5-(p-tolyl)-1H-imidazol-2-yl)piperidin-1-yl)but-3-en-1-on oder eine Mischung davon handelt.

6. Zusammensetzung nach einem der vorhergehenden Ansprüche, wobei es sich bei dem Lösungsmittel (b) um Milchsäure handelt.

7. Zusammensetzung nach einem der vorhergehenden Ansprüche, **dadurch gekennzeichnet, dass** die Zusammensetzung mindestens 3 Gew.-% mindestens einer Verbindung der Formel (I), bezogen auf das Gesamtgewicht der Zusammensetzung, umfasst.

8. Zusammensetzung nach einem der vorhergehenden Ansprüche, im Wesentlichen bestehend aus oder bestehend aus (a) 3-50 Gew.-% 2-Methyl-1-(2-(5-(p-tolyl)-1H-imidazol-2-yl)piperidin-1-yl)butan-1-on und (b) 50-97 Gew.-% Milchsäure, bezogen auf die gesamte Mischung, mit der Maßgabe, dass sich (a) und (b) zu 100 Gew.-% summieren.

9. Duftstoff- oder Geschmacksstoffformulierung, umfassend eine Zusammensetzung gemäß einem der vorhergehenden Ansprüche, ferner umfassend mindestens eine Aktivsubstanz, die aus der Gruppe bestehend aus Geschmacksstoff und Duftstoff ausgewählt ist, und gegebenenfalls Süßungsmittel.

10. Duftstoff- oder geschmacksstoffhaltiges Produkt, umfassend eine Produktbasis und einen eine kühlende Wirkung bereitstellenden Anteil einer Zusammensetzung nach Anspruch 1 oder einer Formulierung nach Anspruch **9.**

11. Verfahren zur Einarbeitung mindestens einer kühlenden Verbindung (a) gemäß Anspruch 1 in ein duftstoff- oder geschmacksstoffhaltiges Produkt, umfassend das Mischen mindestens einer kühlenden Verbindung der Formel (I) mit mindestens einem Lösungsmittel, das aus Butyllactat, Ethanol, Ethyllactat, Isopropylalkohol, Isopropylidenglycerin und Milchsäure oder einer Mischung davon ausgewählt ist, und das Zugeben der resultierenden Zusammensetzung zu dem Produkt.

12. Verfahren zur Bereitstellung einer kühlenden Empfindung für die Haut oder die Schleimhaut, umfassend das Zugeben eines eine kühlende Wirkung bereitstellenden Anteils einer Zusammensetzung nach einem der Ansprüche 1 bis 8 zu einer Produktbasis.

## Revendications

1. Composition comprenant
a) un composé rafraîchissant de formule (I) 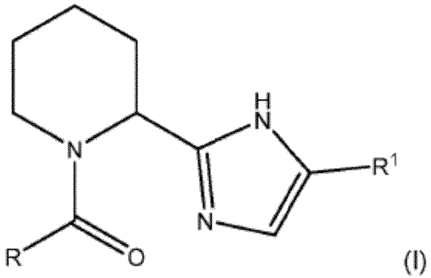
R étant un alkyle ou alcényle ramifié en C₃₋₇ comprenant éventuellement un atome de S, et
R¹ étant phényle, éventuellement substitué par un ou deux ou trois substituants indépendamment choisis dans le groupe constitué par CH₃, F et Cl ; et éventuellement substitué par un autre substituant choisi dans le groupe constitué par Et, vinyle, CN, NO₂, méthoxy et CF₃ ;
b) et au moins un solvant choisi parmi le lactate de butyle, l'éthanol, le lactate d'éthyle, l'alcool isopropylique, l'isopropylidène glycérol et l'acide lactique ou un mélange correspondant.

2. Composition selon la revendication 1, le composé de formule (I) étant représenté par la formule (II) 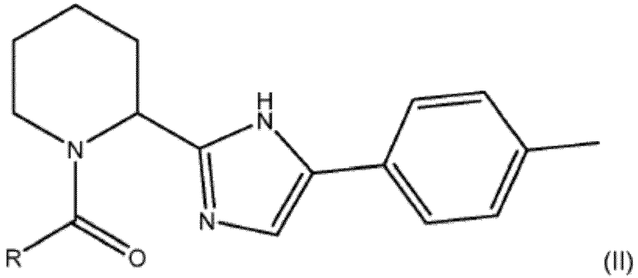 R étant un alkyle ou alcényle ramifié en C₃₋₇ comprenant éventuellement un atome de S.

3. Composition selon la revendication 1 ou la revendication 2, le rapport en poids de l'ingrédient (a) sur le solvant (b) étant de 1 : 20 à 1 : 1.

4. Composition selon une quelconque revendication précédente, la composition étant un liquide.

5. Composition selon une quelconque revendication précédente, le composé rafraîchissant de formule (I) étant 2-méthyl-1-(2-(5-(p-tolyl)-1H-imidazol-2-yl)pipéridin-1-yl)butan-1-one, 2-(méthylthio)-1-(2-(5-(p-tolyl)-1H-imidazol-2-yl)pipéridin-1-yl)propan-1-one, 2-méthyl-2-(méthylthio)-1-(2-(5-(p-tolyl)-1H-imidazol-2-yl)pipéridin-1-yl)propan-1-one, ou 2,2-diméthyl-1-(2-(5-(p-tolyl)-1H-imidazol-2-yl)pipéridin-1-yl)but-3-én-1-one, ou un mélange correspondant.

6. Composition selon une quelconque revendication précédente, le solvant (b) étant l'acide lactique.

7. Composition selon une quelconque revendication précédente, caractérisée ce que la composition comprend au moins 3 % en poids d'au moins un composé de formule (I), par rapport au poids total de la composition.

8. Composition selon une quelconque revendication précédente, essentiellement constituée de, ou constituée de (a) 3 à 50 % en poids de 2-méthyl-1-(2-(5-(p-tolyl)-1H-imidazol-2-yl)pipéridin-1-yl)butan-1-one et (b) 50 à 97 % en poids d'acide lactique, par rapport au mélange total, à condition que (a) et (b) totalisent jusqu'à 100 % en poids.

9. Formulation de fragrance ou d'arôme comprenant une composition telle que définie dans l'une quelconque des revendications précédentes, comprenant en outre au moins un agent actif choisi dans le groupe constitué par un arôme et une fragrance, éventuellement comprenant un agent édulcorant.

10. Produit fragrancé ou aromatisé, comprenant une base de produit et une proportion fournissant un effet rafraîchissant d'une composition selon la revendication 1, ou d'une formulation selon la revendication 9.

11. Procédé d'incorporation d'au moins un composé rafraîchissant (a) tel que défini dans la revendication 1 dans un produit fragrancé ou aromatisé, comprenant le mélange de (a) au moins un composé rafraîchissant de formule (I) avec au moins un solvant choisi parmi le lactate de butyle, l'éthanol, le lactate d'éthyle, l'alcool isopropylique, l'isopropylidène glycérol et l'acide lactique, ou un mélange correspondant, et l'ajout de la composition résultante au produit.

12. Procédé de fourniture à un produit d'une sensation rafraîchissante pour la peau ou la membrane muqueuse, comprenant l'ajout à une base de produit d'une proportion fournissant un effet rafraîchissant d'une composition selon l'une quelconque des revendications 1 à 8.
